# EUROPEAN PATENT APPLICATION

(11) **EP 4 721 643 A1**
(43) Date of publication of application: **08.04.2026**
(21) Application number: 24204670.4
(22) Date of filing: 04.10.2024
(51) Int. Cl.: A61B 3/10, A61B 3/14

(54) **POLARISATION STATE ADJUSTMENT IN AN OPHTHALMIC IMAGING INSTRUMENT**

(71) Applicant: Optos plc, Dunfermline, Scotland KY11 8GR (GB)
(72) Inventor: SWAN, Derek, Dunfermline, Scotland, KY11 8 GR (GB)
(74) Representative: Hoffmann Eitle

(57) **Abstract**

A method of determining an adjustment of an optical element of a widefield ophthalmic imaging instrument operable to image an ocular fundus using non-linearly polarized light whose polarization state is variable by the optical element, comprising: controlling the instrument to acquire each image of a plurality of images of the fundus by adjusting the optical element to set a respective polarization state of the non-linearly polarized light, and controlling the instrument to acquire the image using light of the respective polarization state; analysing each image to determine a respective degree of a variation in at least one of a local contrast or a local brightness across the image; and determining the adjustment of the optical element, based on the degrees of variation and corresponding adjustments to the optical element, such that a fundus image acquired by the instrument after the determined adjustment has been made has less of the variation.

## Description

### [Field]

Example aspects herein generally relate to the field of ophthalmic imaging devices and, in particular, to techniques of adjusting a state of polarization of non-linearly polarized light used by an ophthalmic imaging instrument to image an ocular fundus, and ophthalmic imaging instruments adjusted using these techniques.

### [Background]

Ophthalmic imaging instruments of various kinds are widely used to assess the health of patients' eyes. Improvements in image quality of fundus images have been obtained by using linear polarisers to reduce or eliminate glare caused by specular reflections from surfaces of lenses in the fundus camera (or other fundus imaging instrument being used), as well as from ocular media such as the cornea, intraocular lens and internal limiting membrane of the retina. These specular reflections can impair the visibility of fundus features and thus reduce the quality of fundus images. Another widely used approach to suppressing glare and other image artifacts is use of circular polarisers. When a beam of circularly polarised light is reflected by a smooth surface, the handedness of the circular polarisation is reversed, so that a right-circularly polarised light becomes left-circularly polarised, for example. The reflected beam can be blocked by a right-circular polariser in the return beam path, resulting in improved fundus image quality. Separating the paths of illumination and observation is another approach to suppressing glare. It is also known to combine these approaches, by placing a respective circular polariser in each of the illumination path and the observation path.

### [Summary]

There is provided, in accordance with a first example aspect herein, a method of determining an adjustment to be made to an optical element of an ophthalmic imaging instrument, the ophthalmic imaging instrument being operable to image a region of an ocular fundus by illuminating the region with non-linearly polarized light and detecting light from the illuminated region, the optical element being adjustable to vary a state of polarization of the non-linearly polarized light. The method comprises controlling the ophthalmic imaging instrument to acquire each image of a plurality of images of the region by making a respective adjustment to the optical element to set a respective state of polarization of the non-linearly polarized light, and controlling the ophthalmic imaging instrument to illuminate the region with light of the respective state of polarization and acquire the image by detecting light from the illuminated region. The method further comprises analysing each image of the plurality of images to determine a respective degree of a variation in at least one of a local contrast or a local brightness across a representation of the region in the image. The method further comprises determining the adjustment to be made to the optical element, based on the degrees of variation determined by analysing the images and corresponding adjustments made to the optical element to acquire the images, the determined adjustment being such that an image of the region acquired by the ophthalmic imaging instrument after the determined adjustment has been made has less of the variation. The method may further comprise making the determined adjustment such that an ocular fundus image acquired after the determined adjustment has been made has less variation in the at least one of the local contrast or the local brightness across the ocular fundus image.

In some example embodiments, the adjustment to be made to the optical element may be determined to minimise the variation in the at least one of the local contrast or the local brightness across the representation of the region in the image acquired by the ophthalmic imaging instrument after the determined adjustment has been made. In other example embodiments, the adjustment to be made to the optical element may be determined to reduce the variation in the at least one of the local contrast or the local brightness across the representation of the region in the image acquired by the ophthalmic imaging instrument after the determined adjustment has been made to below a predetermined level.

In the first example aspect or any of the example embodiments above, the ophthalmic imaging instrument may, in accordance with an example embodiment, further comprise a polarised light source arranged to generate linearly polarised light, the optical element may comprise a quarter-wave plate arranged to generate the non-linearly polarized light from the linearly polarised light, and an angle of rotation of the quarter-wave plate about a propagation direction of the linearly polarised light may be adjusted to set the respective state of polarization of the non-linearly polarized light for acquiring each image of the plurality of images.

In the first example aspect or any of the example embodiments above, the determined adjustment, when made to the optical element, may cause the ophthalmic imaging instrument to illuminate an imaging target being imaged by the ophthalmic imaging instrument with circularly polarised light. Furthermore, the non-linearly polarized light may have at least one wavelength between 400 nm and 570 nm. The ophthalmic imaging instrument may be a widefield (WF) ophthalmic imaging instrument or an ultra-widefield (UWF) ophthalmic imaging instrument. The ophthalmic imaging instrument may comprise at least one of a WF/UWF fundus camera, a WF/UWF scanning laser ophthalmoscope or a WF/UWF optical coherence tomography instrument.

There is also provided, in accordance with a second example aspect herein, a computer program which, when executed by a processor arranged to control a ophthalmic imaging instrument, which is operable to image a region of an ocular fundus by illuminating the region with non-linearly polarized light whose state of polarisation is variable by adjustment of an optical element, cause the processor to perform the method according to the first example aspect or any of the example embodiments set out above.

There is also provided, in accordance with a third example aspect herein, a non-transitory computer-readable storage medium storing the computer program according to the second example aspect herein.

There is provided, in accordance with a fourth example aspect herein, an ophthalmic imaging instrument operable to image a region of an ocular fundus by illuminating the region with non-linearly polarized light and detecting light from the region, the ophthalmic imaging instrument comprising an optical element which is adjustable to vary a state of polarization of the non-linearly polarized light, wherein the optical element has been adjusted to be in a state of adjustment such that further adjustment of the optical element to vary the state of polarization of the non-linearly polarized light causes a degree of a variation in at least one of a local contrast or a local brightness across a representation of the region in a subsequent image acquired by the ophthalmic imaging instrument after the further adjustment to be at least as large as a degree of the variation in at the least one of the local contrast or the local brightness across a representation of the region in an initial image acquired by the ophthalmic imaging instrument whilst the optical element is in the state of adjustment.

The ophthalmic imaging instrument may further comprise a polarised light source arranged to generate linearly polarised light, the optical element may comprise a quarter-wave plate arranged to generate the non-linearly polarized light from the linearly polarised light, and an angle of rotation of the quarter-wave plate about a propagation direction of the linearly polarised light may be adjustable to vary the state of polarization of the non-linearly polarized light. Additionally or alternatively, the state of adjustment of the optical element may be such that the region is illuminated with circularly polarized light during imaging of the region by the ophthalmic imaging instrument. The non-linearly polarized light may have at least one wavelength between 400 nm and 570 nm. Furthermore, the ophthalmic imaging instrument may be a widefield (WF) ophthalmic imaging instrument or an ultra-widefield (UWF) ophthalmic imaging instrument, and may comprise at least one of a WF/UWF fundus camera, a WF/UWF scanning laser ophthalmoscope, or a WF/UWF optical coherence tomography instrument.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ultra-widefield ophthalmic imaging instrument according to an example embodiment herein, which is operable to image a fundus of an eye using non-linearly polarized light whose state of polarisation can be set by adjustment of an optical element.
Figure 2 is a schematic illustration of a first optical arrangement for generating elliptically polarised light, which comprises a rotatable quarter-wave plate as an example of the optical element of the example embodiment.
Figure 3 is a schematic illustration of a second optical arrangement for generating elliptically polarised light, which comprises a rotatable linear polariser as another example of the optical element of the example embodiment.
Figure 4 is a schematic illustration of an example of a scanning system forming part of the ophthalmic imaging instrument of the example embodiment.
Figure 5 is a flow diagram illustrating a method according to an example embodiment, by which an adjustment to be made to the optical element of the ultra-widefield ophthalmic imaging instrument of Figure 1 is determined.
Figure 6 is a schematic illustration of a programmable signal processing hardware, which may be configured to perform the at least some of the processes of the methods described herein.

### [Detailed Description of Example Embodiments]

In brief, the inventors have found that an improvement in image quality of widefield (WF) and ultra-widefield (UWF) retinal images, specifically in terms of an increased uniformity in local contrast and/or local brightness across a WF/UWF image as a whole, can be obtained by illuminating the retina with non-linearly polarised light having a state of polarisation typically close to circular, and tuning the state of polarisation of the non-linearly polarised light to maximise or at least increase the image uniformity. The polarisation state adjustment techniques described herein can improve the utility of many conventionally adjusted ophthalmic imaging instruments.

In the context of WF/UWF retinal imaging, the use of non-linearly polarised light may provide advantages in terms of an increase in the uniformity of both the light delivered to the retina by the ophthalmic imaging instrument, and the light reflected by the retina.

Regarding the uniformity of the light delivered to the retina, in a scanning WF/UWF ophthalmic imaging instrument, the imaging beam may be subject to relatively high angles of reflection or refraction as the beam propagates through the instrument. Moreover, the great variation in these angles that typically occurs during a scan may give rise to variations in the intensity of the reflected/refracted light. Linearly polarised light is susceptible to a larger variation in intensity as a function of the angle of incidence on the optical components as compared to non-linearly polarised light. Accordingly, the intensity of linearly polarised light delivered to the retina tends to have a larger variation across the retina than the intensity of non-linearly polarised light.

Regarding the light reflected by the retina, its intensity is generally more uniform across the scanned region of the retina when the illumination has non-linear polarisation than where linearly polarised light is used to illuminate the retina. Non-linearly polarised light is less susceptible to variations in the patient's retina that may affect the intensity of the light reflected by the retina. The illumination provided during a retinal scan tends to interact mainly with the upper layers of the retina (e.g. the retinal nerve fiber layer, RNFL), which is typically thickest around the optic nerve head and thins towards the periphery. The effect is more pronounced for shorter wavelengths (e.g. as provide by a blue or green laser). The reduction in the thickness of the RNFL generally causes a more pronounced reduction in intensity of reflected light where the imaging beam is linearly polarised as compared to the case where the beam is non-linearly polarised. Non-linearly polarised light is thus less susceptible to variations in thickness of the retina, thereby resulting in a more uniform intensity of light being reflected by the retina.

From a clinician's perspective, a WF/UWF retinal image having less variation in local contrast and/or local brightness across it can be assessed more reliably as compared to one having more of the variation. For example, pathologies located in a darker region of a WF/UWF retinal image may be harder to recognise (whether by a human observer or an algorithm used in retinal image processing), thereby making it more difficult to assess the ocular health of a patient. A WF/UWF retinal image of more uniform contrast/brightness may be assessed more reliably, as all the imaged regions of the retina have similar contrast/brightness.

Example embodiments herein will now be described in detail with reference to accompanying drawings.

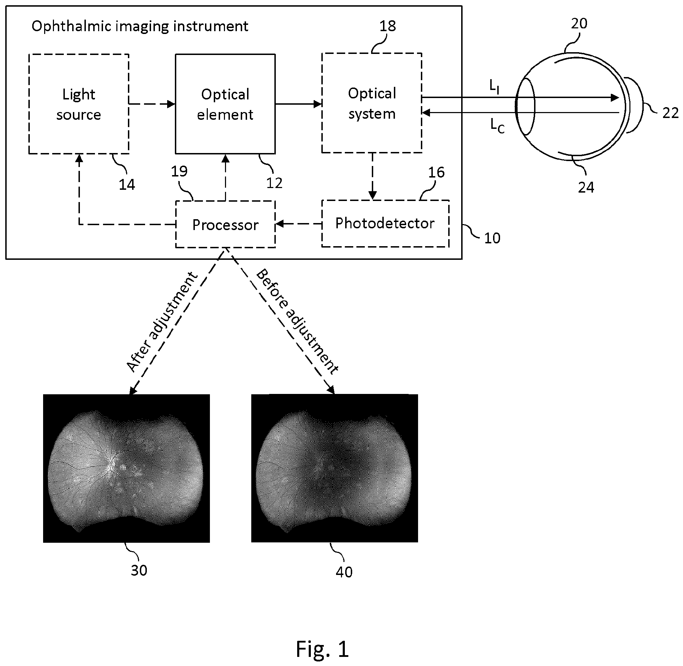

Figure 1 is a schematic illustration of an ophthalmic imaging instrument 10, which is operable to image a portion of an eye 20 of a subject, the portion comprising a region 22 of an ocular fundus 24 of the eye 20. The ocular fundus 24 is the inner lining of the eye 20 opposite the lens, and comprises the retina, macula, optic disc, fovea, choroid and blood vessels. The ophthalmic imaging instrument 10 may be further operable to image another portion of the eye 20, such as at least a part of the anterior segment of the eye 20.

The ophthalmic imaging instrument 10 may be a widefield (WF) ophthalmic imaging instrument, which is operable to acquire a widefield image of the fundus 24. A WF image of the fundus 24 is defined as a single-capture image, centred on the fovea of the eye 20, which captures retinal anatomic features in the mid-periphery that are posterior to the vortex vein ampulla, in all four quadrants (i.e. superior, inferior, nasal and temporal). A WF ophthalmic imaging instrument can thus acquire a single-capture image which covers a region of the retina extending from the fovea up to the posterior edge of vortex vein ampulla, and is defined to have a field of view (FoV) between 60 degrees and 100 degrees. Here, the FoV is expressed in terms of the eye-angle, which is the angle subtended by the imaged retinal region at the spherical centre of the eye, i.e. the point of intersection between the vertical diameter of the eyeball and the visual axis.

The ophthalmic imaging instrument 10 may, as in the present example embodiment, be provided in form of an ultra-widefield (UWF) ophthalmic imaging instrument, which has a larger FoV than a WF instrument and is operable to acquire an UWF image of the fundus 24 covering a larger proportion of the ocular fundus 24. An UWF image of the ocular fundus 24 is defined as a single-capture image, centred on the fovea of the eye 20, which captures retinal anatomic features in the far periphery that are anterior to the vortex vein ampulla in all four quadrants. An UWF ophthalmic imaging instrument can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so - called Optomap^{™} images) covering up to 200 degrees of the fundus (or approximately 82% of the retina) in a single capture.

Although improvements in image quality resulting from the use of the techniques described herein are particularly apparent in WF and UWF images captured by WF and UWF ophthalmic imaging instruments, respectively, they may also be observed in images captured by ophthalmic imaging instruments having a smaller FoV.

In the present example embodiment, the ophthalmic imaging instrument 10 comprises a multi-modal UWF scanning laser ophthalmoscope (SLO), which is capable of imaging in two or more different imaging modalities. Examples of such imaging modalities include pseudo-colour imaging, fundus autofluorescence (FAF), fluorescein angiography (FA), and indocyanine green angiography (ICGA). However, the ophthalmic imaging instrument 10 need not be multi-modal, and may instead be capable of operating in only one of the aforementioned imaging modalities or another imaging modality. Furthermore, the form of the ophthalmic imaging instrument 10 is not limited to SLO, and the ophthalmic imaging instrument 10 may additionally or alternatively comprise a fundus camera or an optical coherence tomography (OCT) instrument, for example. By way of an example, the Optos Monaco^{™} is a combined SLO-OCT system capable of acquiring green or red (or combined green and red) laser views, green laser autofluorescence views, and OCT views.

The ophthalmic imaging instrument 10 is operable to image the region 22 of the ocular fundus 24 by illuminating the region 22 with non-linearly polarized light L_{I} and collecting and detecting a portion of the light illuminating the region 22 that has been scattered or reflected from the region 22. The non-linearly polarized light L_{I} may include or consist of circularly polarised light or elliptically polarised light. The state of polarization of the non-linearly polarized light L_{I} is variable by means of an adjustable optical element 12, which forms part of the ophthalmic imaging instrument 10.

The state of polarisation of the non-linearly polarised light L_{I} may be set by making an adjustment to the optical element 12 which put the optical element 12 in a corresponding state of adjustment.

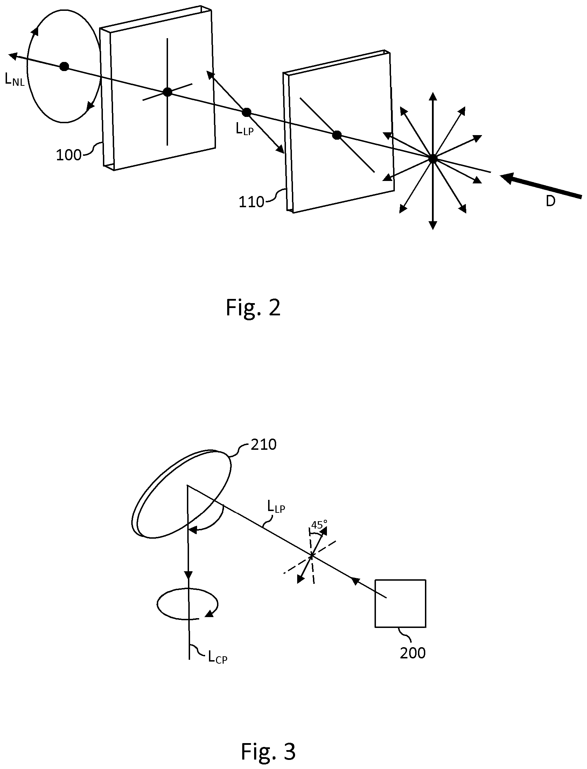

For example, the optical element 10 may, as in the present example embodiment, comprise a rotatable quarter-wave plate 100 as illustrated schematically in Figure 2, which is arranged to generate non-linearly polarised light L_{NL} from linearly polarised light L_{LP} normally incident on the quarter-wave plate 100 from a polariser 110, wherein an angle of rotation of the quarter-wave plate 100 about a propagation direction D of the linearly polarised light L_{LP} is adjustable manually or by any suitable rotation mechanism to set the state of polarization of the non-linearly polarized light L_{I} incident on the fundus 24 for acquiring an image of the fundus 24 by the ophthalmic imaging instrument 10. For example, passing linearly polarised light L_{LP} from the polariser 110 through the quarter-waveplate 100 with its fast and slow axes at ±45 degrees to the polarisation axis of the linearly polarised light L_{LP} will convert the linear polarisation to a circular polarisation, as illustrated in Figure 2. Rotating the axes of the quarter-waveplate 100 relative to the plane of polarisation of the linearly polarised light L_{LP} away from the 45-degree angle (but not enough to cause the linearly polarised light L_{LP} to be parallel to the slow or fast axis of the quarter-wave plate 100), and/or having a quarter-wave plate whose thickness is not matched to the wavelength of the light being used precisely enough, will cause elliptically polarised light to emerge from the quarter-wave plate 100. The quarter-wave plate 100 may be formed of any suitable birefringent material that is well-known for this application, including but not limited to quartz, mica or a polymer (e.g. liquid crystal polymer), for example, and may be a multiple-order waveplate or a zero-order waveplate, for example.

As another example, the optical element 10 may, as illustrated schematically in Figure 3, comprise a rotatable linear polariser 200, via which a beam of light propagates to a quarter wave phase-shifting mirror 210 (also widely referred to as a quarter wave phase retarder mirror or a lambda/4 mirror) that may also form part of the ophthalmic imaging instrument 10. In case the phase-shifting mirror 210 is arranged to receive a beam of linearly polarised light L_{LP} from the linear polariser 200 at an incidence angle of 45 degrees relative to the normal of the mirror's surface, and the plane of polarisation of the linearly polarised light L_{LP} is at an angle of 45 degrees relative to a plane containing the beam of the linearly polarised light an a reflected beam L_{R} which is a reflection of the incident linearly polarised beam L_{LP} by the phase-shifting mirror 210, the reflected beam L_{R} will be circularly polarised. An angle of rotation of the linear polariser 200 about a propagation direction of the beam of light may be adjusted manually or by any suitable rotation mechanism to set the state of polarization of the non-linearly polarized light L_{R} from the phase-shifting mirror 210 for acquiring an image by the ophthalmic imaging instrument 10. Rotating the linear polariser 200 away from the 45-degree angle mentioned above, and/or changing the angle of incidence of the linearly polarised light beam L_{LP} on the phase-shifting mirror 210, will cause the reflected beam L_{R} to be elliptically polarised.

Referring again to Figure 1, the ophthalmic imaging instrument 10 may, as in the present example embodiment, comprise a light source 14, a photodetector 16, an optical system 18 and a processor (or controller) 19 in addition to the optical element 12 and any of the further components mentioned above.

The optical system 18 is arranged to illuminate the region 22 of the ocular fundus 24 with non-linearly polarised light L_{I}, which the optical element 12 generates from light from the light source 14, and to collect light, L_{C}, from the illuminated region 22. The optical system 18 is further arranged to convey or guide (using one or more optical components, such as optical fibres, lenses, mirrors and the like) the collected light Lc towards the photodetector 16.

The light source 14 is, in general, arranged to generate light in one or more ranges of wavelength that are suitable for imaging the ocular fundus 24, for example in the visible spectrum (e.g. red and green light) and/or the near-infrared spectrum. The light generated by the light source 14 preferably has at least one wavelength between 400 nm and 570 nm, as the polarising nature of certain shallow retinal structures (notably the retina nerve fibre layer, RNFL) that are often of interest to observe tends to improve the visibility of these structures when imaged with circularly polarised (or, more generally, non-linearly polarised) light. The light source 14 may, for example, comprise one or more laser diodes or super-luminescent diodes (or a combination of laser diodes or super-luminescent diodes), and may also have one or more optical components (such as collimators, apertures, lens) arranged to generate one or more light beams. By way of an example, the light source 14 of the present example embodiment comprises a green laser arranged to generate green light (e.g. of 532 nm wavelength). The optical system 18 (discussed further below) may be arranged to illuminate the region 22 of the ocular fundus 24 with a (flying) spot of light or a line of light (in case of the ophthalmic imaging instrument 10 being a line-field system) generated using a cylindrical lens or other well-known components or optical assemblies for generating line-field illumination.

In example embodiments wherein the ophthalmic imaging instrument 10 is operable in an OCT imaging mode, the light source 14 may comprise a swept light source (in case of the ophthalmic imaging instrument 10 being a swept-source OCT (SS-OCT) system) or a broadband source (in case of the ophthalmic imaging instrument 10 being a spectral-domain OCT (SD-OCT) system).

The form of the photodetector 16 is not limited and it may, for example, comprise a balanced photodetector arrangement comprising two reverse-biased photodiodes whose output photocurrents are subtracted from one another, the subtracted current signal being converted to a voltage detection signal by a transimpedance amplifier. In example embodiments where the ophthalmic imaging instrument 10 features an OCT imaging modality, the photodetector 16 may comprise a spectrometer (where a SD-OCT set-up is used) or a photodiode detector (where a SS-OCT set-up is used).

The optical system 18 may, as in the present example embodiment, comprise a scanning system, which is arranged to perform a two-dimensional point-scan of the non-linearly polarised light L_{I} from the optical element 12 across a region 22 of the ocular fundus 24 that is illuminated by the point-scan, and to collect light from the illuminated region 22 during the point-scan.

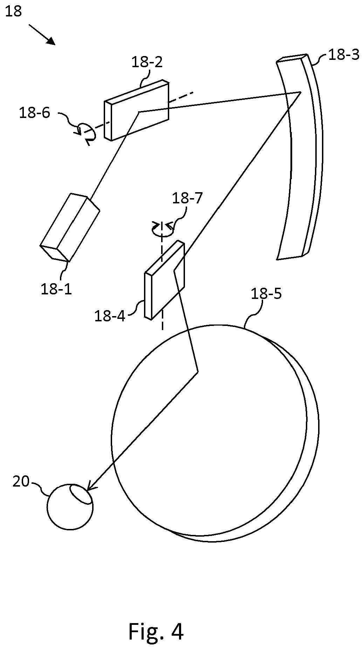

An example of such an optical system is illustrated in Figure 4. The optical system 18 may, as in the present example embodiment, comprise a scanning element and a mirror, wherein the optical system 18 is arranged to perform the two-dimensional point scan by the scanning element scanning a light beam across the region 22 via the mirror. An example of such a scanning system, which can perform a WF or UWF retinal scan, is described in WO 2014/53824 A1, the content of which is incorporated herein by reference in its entirety. Components of such a scanning system are shown in Figure 4 and comprise an optical coupler 18-1, a first scanning element 18-2, a first curved mirror 18-3, a second scanning element 18-4 and a second curved mirror 18-5. The light beam enters the optical system 18 via the optical coupler 18-1. The light beam is then reflected, in sequence, by the first scanning element 18-2, the first curved mirror 18-3, the second scanning element 18-4 and the second curved mirror 18-5, before being incident on the eye 20. Light from the illuminated region 22 of the ocular fundus 24 collected by the optical system 18 follows the same optical path through the optical system 18 as the light beam that had entered the optical system 18 via the optical coupler 18-1 but in reverse order, and exits the optical system 18 via the optical coupler 18-1.

The two-dimensional point scan is performed by the first scanning element 18-2 rotating around a first axis 18-6 to scan the light beam in a first direction across the region 22, and by the second scanning element 18-4 rotating around a second axis 18-7 to scan the light beam in a second direction across the region 22 (which may, as in the present example embodiment, be orthogonal to the first direction). Thus, by rotating the first scanning element 18-2 and the second scanning element 18-4, it is possible to steer the light beam to different locations on the region 22 of the ocular fundus 24. The rotation of the first scanning element 18-2 and the second scanning element 18-4 may be coordinated by a scanning system controller (not shown) such that the light beam is scanned across the region 22 in accordance with a predefined scan pattern.

In the example of Figure 4, the first curved mirror 18-3 is an ellipsoidal mirror (and referred to as a slit mirror), and the second curved mirror 18-5 is also an ellipsoidal mirror. Each of the ellipsoidal mirrors has a respective first focal point and a respective second focal point. The first scanning element 18-2 is disposed at the first focal point of the first curved mirror 18-3, and the second scanning element 18-4 is disposed at the second focal point of the first curved mirror 18-3. The second scanning element 18-4 is also disposed at the first focal point of the second curved mirror 18-5, and the eye 20 (more specifically, the pupil of the eye 20 in the present example) is disposed at the second focal point of the second curved mirror 18-5.

In the present example embodiment, the first scanning element 18-2 takes the form of a polygon scanning mirror, while the second scanning element 18-4 takes the form of a galvanometer optical scanner (or "galvo"). However, the first scanning element 18-2 second scanning element 18-4 may be provided in other forms, such as a MEMS scanning mirror or a resonant scanning mirror, for example.

An optical system 18 of the kind described above with reference to Figure 4 may be used in a variety of imaging modes, such as colour, red-free, AF, ICG and OCT (among others). However, the optical system 18 may alternatively be arranged in other ways, for example to instead perform a line-field scan across the imaged region 22 of the ocular fundus 24. The optical system 18 may further comprise additional optical components, such as optical components for filtering, linearly/circularly polarising, guiding and/or collimating the light from the light source 14, and a cylindrical lens or other means of generating a line of light in case the optical system 18 is a line-field system. For example, the polariser 110 shown in Figure 2 may be provided as a stand-alone component of the optical system 18 or the ophthalmic imaging instrument 10 or alternatively as an intrinsic part of the light source 14 in the present example embodiment. The light source 14 may thus natively output linearly polarised light in some cases. In other cases, the light source 14 may be arranged to emit randomly polarised light, which is then linearly polarised by an external linear polariser; in such cases, the combination of the light source and external linear polariser can be considered to provide a polarised light source. In other example embodiments, which include the arrangement of Figure 3, the quarter wave phase-shifting mirror 210 may form part of the optical system 18 or the ophthalmic imaging instrument 10.

It should be noted that the mirror-based ophthalmic imaging instrument 10 described above has been given by way of an example only, and that an ophthalmic imaging instrument according to other example embodiments may employ a lens-based optical system instead of a mirror-based one of the kind described with reference to Figure 4, with one or more lenses arranged to light-guiding functions of mirrors 18-3 and 18-5.

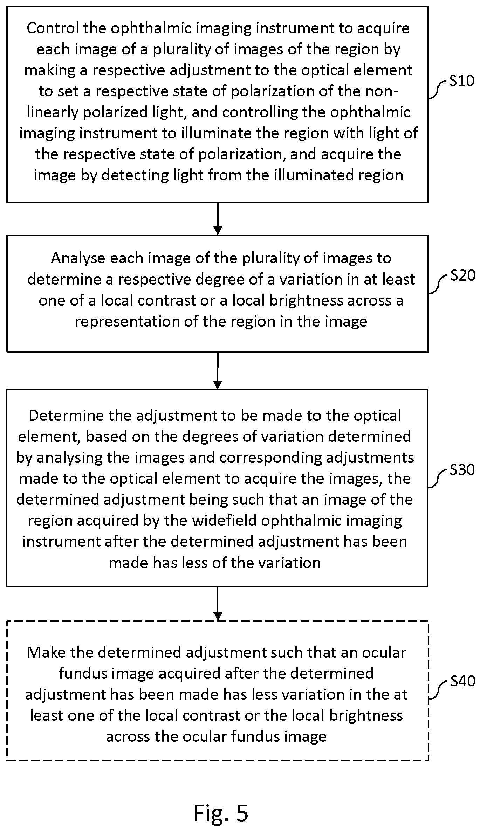

Figure 5 is a flow diagram illustrating a method of determining an adjustment to be made to the optical element 12 of the ophthalmic imaging instrument 10 of the example embodiment described above, which adjustment results in an image 30 of the region 22 acquired by the ophthalmic imaging instrument 10 after the determined adjustment has been made having less variation in a local contrast and/or a local brightness across a representation of the region 22 in the image 30 than an image 40 of the region 22 acquired before the adjustment was made, as illustrated in Figure 1. The determined adjustment may seek to minimise the variation on the local contrast and/or local brightness in fundus images acquired by the ophthalmic imaging instrument 10. This method may be performed solely by the processor 19, as in the present example embodiment, or only partially by the processor 19, with some or more of the process steps being performed manually by a human operator of the ophthalmic imaging instrument 10. The processor 19 generates images 30 and 40 (among others) based on a detection output of the photodetector 16 using well-known techniques.

The image 30 shown in Figure 1 provides a schematic illustration of how the determined adjustment to the optical element 12 may reduce the variation in a local contrast and/or a local brightness across a representation of the region 22 in the image, thus making the image 30 more uniform in terms of local contrast and/or brightness than image 40. However, the reduction in the variation is more subtle in reality, and it is difficult to show clearly in the present patent document. In practice, the reduction in the variation can usually be observed using high-resolution image viewers of the kind typically employed by clinicians in their analysis of WF/UWF images, and is found to have a degree of variation among patients, typically being more prominent where the fundus has a relatively high reflectivity.

In process S10 of Figure 5, the processor 19 controls the ophthalmic imaging instrument 10 to acquire a plurality of images of the region 22, with each image of the plurality of images being acquired by firstly making a respective adjustment to the optical element 12 to set a respective state of polarization of the non-linearly polarized light L_{I}, and then controlling the ophthalmic imaging instrument 10 to illuminate the region 22 with light of the respective state of polarization, and to acquire the image by detecting light Lc from the illuminated region 22. The respective state for polarisation set for each image is thus different to the state of polarisation set for every other image of the plurality of images of the region 22, while all other imaging parameters remain the same throughout the acquisition of the images in S10. By way of an example, where the optical element 12 comprises a rotatable quarter-wave plate, as in the present example embodiment, the angle of rotation of the quarter-wave plate about the propagation direction of the linearly polarised light L_{LP} is adjusted under the control of processor 19 in S10 of Figure 5 to set the respective state of polarization of the non-linearly polarized light L_{I} for acquiring each image of the plurality of images. Between image acquisitions performed by the ophthalmic imaging instrument 10 in process S10, the quarter-wave plate may be rotated by a rotational drive mechanism, which is under the control of the processor 19, in regular or irregular angular increments, which may be within a range from a fraction of a degree to a few degrees (e.g. 0.5 to 2 degrees). A plurality of fundus images is thus acquired, with each image having been acquired using light of a different state of non-linear polarisation incident on the fundus 24.

In process S20 of Figure 5, the processor 19 analyses each image of the plurality of images to determine a respective degree of a variation in a local contrast and/or a local brightness across a representation of the region 22 in the image. The processor 19 may do this in one of several different ways.

For example, the processor 19 may divide the representation of the region 22 in the image into image tiles, and calculate the respective standard deviation (SD) or variance of intensity values of the pixels in each tile as a respective value of local image contrast for the tile. The processor 19 may then calculate the variance of these SD/variance values, for example, to measure the degree of variation in local contrast across the representation of the region 22 in the image.

The processor 19 may additionally or alternatively determine a respective degree of a variation in a local brightness across the representation of the region 22 in the image. The processor 19 may evaluate the variation in local image brightness in one of several different ways. For example, the processor 19 may likewise divide the representation of the region 22 in the image into image tiles, and calculate the respective mean of intensity values of the pixels in each tile as a respective value of local image brightness for the tile. The processor 19 may then calculate the variance of these mean values, for example, to measure the degree of variation in local brightness across the representation of the region 22 in the image.

In process S30 of Figure 5, the processor 19 determines the adjustment to be made to the optical element 12, based on the degrees of variation determined in process S20 by analysing the images and information on the corresponding adjustments which were made to the optical element 12 to acquire the images, such that an image 30 of the region 22 acquired by the ophthalmic imaging instrument 10 after the determined adjustment has been made has less of the aforementioned variation.

For example, the processor 19 may fit an appropriate function to data indicating how the determined degrees of variation change with increasing rotation of the quarter-wave plate (or, more generally, with progressive adjustment of the optical element 12), and then find an extremum in the fitted function which indicates an optimal amount of rotation of the quarter-wave plate (or, more generally, an optimum adjustment of the optical element 12) that would minimise the variation in local contrast or local image brightness across an image acquired by the ophthalmic imaging instrument 10, making the image appear more uniform to an observer and facilitating inspection of anatomical features that have been imaged across the image.

As an alternative, the processor 19 may identify in process S30 an adjustment made in process S10 which resulted in the smallest variation in local contrast or local brightness (as the case may be) that was determined in process S20. The adjustment to be made to the optical element 12 may thus be determined to minimise the variation in the local contrast or the local brightness across the representation of the region 22 in the image 30 acquired by the ophthalmic imaging instrument 10 after the determined adjustment has been made. In some cases, the variation in local contrast or local brightness (as the case may be) that was determined in process S20 may be substantially the same for a range of the adjustments to the optical element 12. In such cases, the processor 19 may identify two ends of the range and select an adjustment within the range (e.g. at the midpoint of the range) as the adjustment which is to be made to the optical element 12.

In some example embodiments, it may suffice for the adjustment to be made to the optical element 12 to simply reduce, to below an acceptable level, the variation in the local contrast or the local brightness across the representation of the region 22 in an image 30 acquired by the ophthalmic imaging instrument 10 after the determined adjustment has been made. In these example embodiments, the processor 19 may firstly compare the degree of variation determined for each adjustment of the optical element 12 with a predetermined threshold to determine whether the threshold is exceeded and, if so, to discard the adjustment from further processing and otherwise to retain the adjustment for further processing. For adjustments which have been retained, the processor 19 may determine, as the adjustment which is to be made to the optical element 12, an adjustment which is representative of the retained adjustments, for example a mean of median of the retained adjustments.

In optional process S40 of Figure 5, the processor 19 makes the determined adjustment such that an ocular fundus image acquired after the determined adjustment has been made has less variation in the local contrast and/or the local brightness across the ocular fundus image. The processor 19 may, as in the present example embodiment, make the determined adjustment to the optical element 12 of the ophthalmic imaging instrument 12 such that the image 30 acquired after the determined adjustment has been made has less variation in the local contrast and/or the local brightness across the image 30. The method of Figure 5 may thus be method of not only determining the adjustment to be made to the optical element 12 but also making the determined adjustment to the optical element 12. The determined adjustment, when made to the optical element 12, may cause the ophthalmic imaging instrument 10 to illuminate an eye of other imaging target being imaged by the ophthalmic imaging instrument 11 with circularly polarised light. This was found to substantially reduce variations in local contrast and local image brightness within acquired images, making the images appear more uniform. It should be noted that, although the determined adjustment of the optical element 12 may cause the eye 20 (or other imaging target) to be illuminated with circularly polarised light, the non-linearly polarised light generated by the optical element (e.g. the beam of non-linearly polarised light L_{NL} emerging from the rotatable quarter-wave plate 100 of Figure 2) will generally not be circularly polarised but will become so after passing through the optical system 18, whose optical components may affect the phase relation between the two orthogonal electric field component vectors of the light.

In cases where the optical element 12 has been adjusted in process S40 to be in a state of adjustment which minimises the aforementioned variation, further adjustment of the optical element 12 to vary the state of polarization of the non-linearly polarized light L_{I} may cause a degree of a variation in the local contrast and/or local brightness across a representation of the region 22 in a subsequent image acquired by the ophthalmic imaging instrument 10, after the further adjustment has been made, to be at least as large as a degree of the variation in an initial image acquired by the ophthalmic imaging instrument 10 whilst the optical element 12 is in the state of adjustment.

However, the adjustment determined in process S30 of Figure 5 may have further utility, particularly in being suitable for adjusting the control element of a duplicate of the ophthalmic imaging instrument 10. Accordingly, in an industrial application where sufficiently similar duplicates of the ophthalmic imaging instrument 10 have been manufactured, the performance of processes S10 to S30 using one duplicate of the ophthalmic imaging instrument 10 may yield an adjustment that can be made to the optical element 12 of any other duplicate of the ophthalmic imaging instrument 10 in order to improve its image quality as described above.

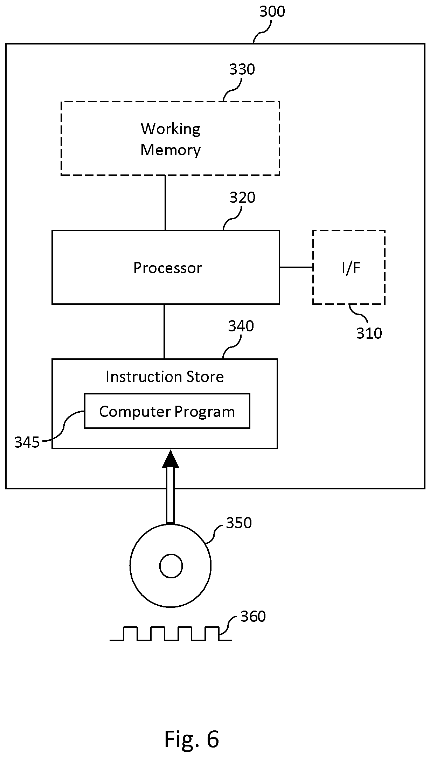

The processor 19 may be provided in any suitable form, for example as a processor 320 of a programmable signal processing hardware 300 of the kind illustrated schematically in Figure 6. The programmable signal processing hardware 300 comprises a communication interface (I/F) 310, for sending control signals to a drive mechanism (where provided) for adjusting the optical element 12 (e.g. rotating the rotatable quarter-wave plate 100 or the rotatable linear polariser 200, as described above), and further control signals to other components of the ophthalmic imaging instrument 10 for controlling it to illuminate the region 22 with light of the different states of polarization, and acquire images by detecting light Lc from the illuminated region 22. The communication interface (I/F) 310, may further serve to receive images acquired by the ophthalmic imaging instrument 10, and send control signals for making the determined adjustment to the optical element 12 via the drive mechanism (where provided). The signal processing hardware 300 further comprises a processor 320 (e.g. a Central Processing Unit, CPU, and/or a Graphics Processing Unit, GPU), a working memory 330 (e.g. a random-access memory) and an instruction store 340 storing a computer program 345 comprising the computer-readable instructions which, when executed by the processor 320, cause the processor 320 to perform various functions of the processor 19 described herein.

The working memory 330 stores information used by the processor 320 during execution of the computer program 345, including intermediate processing results in processes S20 and S30 described above. The instruction store 340 may comprise a ROM (e.g. in the form of an electrically erasable programmable read-only memory (EEPROM) or flash memory) which is pre-loaded with the computer-readable instructions. Alternatively, the instruction store 340 may comprise a RAM or similar type of memory, and the computer-readable instructions of the computer program 345 can be input thereto from a computer program product, such as a non-transitory, computer-readable storage medium 350 in the form of a CD-ROM, DVDROM, etc. or a computer-readable signal 360 carrying the computer-readable instructions. In any case, the computer program 345, when executed by the processor 320, causes the processor 320 to perform the functions of the processor 19 as described herein. In other words, the processor 19 of the present example embodiment may comprise a computer processor 320 and a memory 340 storing computer-readable instructions which, when executed by the computer processor 320, cause the computer processor 320 to perform the functions of the processor 19 as described herein.

It should be noted, however, that the processor 19 may alternatively be implemented in non-programmable hardware, such as an ASIC, an FPGA or other integrated circuit dedicated to performing the functions of the processor 19 described herein, or a combination of such non-programmable hardware and programmable hardware as described above with reference to Figure 5.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the processor 19, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the processor 19 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A method of determining an adjustment to be made to an optical element (12) of an ultra-widefield ophthalmic imaging instrument (10) which is operable to image a region (22) of an ocular fundus (24) by illuminating the region (22) with non-linearly polarized light (L_{I}) and detecting light from the illuminated region (22), the optical element (12) being adjustable to vary a state of polarization of the non-linearly polarized light (L_{I}), the method comprising:
controlling (S10) the ultra-widefield ophthalmic imaging instrument (10) to acquire each image of a plurality of images of the region (22) by:
making a respective adjustment to the optical element (12) to set a respective state of polarization of the non-linearly polarized light (L_{I}); and
controlling the ultra-widefield ophthalmic imaging instrument (10) to illuminate the region (22) with light of the respective state of polarization, and acquire the image by detecting light (Lc) from the illuminated region (22);
analysing (S20) each image of the plurality of images to determine a respective degree of a variation in at least one of a local contrast or a local brightness across a representation of the region (22) in the image; and
determining (S30) the adjustment to be made to the optical element (12), based on the degrees of variation determined by analysing the images and corresponding adjustments made to the optical element (12) to acquire the images, the determined adjustment being such that an image (30) of the region (22) acquired by the ultra-widefield ophthalmic imaging instrument (10) after the determined adjustment has been made has less of the variation.

2. The method according to Claim 1, further comprising making the determined adjustment (S40) such that an ocular fundus image acquired after the determined adjustment has been made has less variation in the at least one of the local contrast or the local brightness across the ocular fundus image.

3. The method according to Claim 1 or Claim 2, wherein the adjustment to be made to the optical element (12) is determined to minimise the variation in the at least one of the local contrast or the local brightness across the representation of the region (22) in the image (30) acquired by the ultra-widefield ophthalmic imaging instrument (10) after the determined adjustment has been made.

4. The method according to Claim 1 or Claim 2, wherein the adjustment to be made to the optical element (12) is determined to reduce the variation in the at least one of the local contrast or the local brightness across the representation of the region (22) in the image (30) acquired by the ultra-widefield ophthalmic imaging instrument (10) after the determined adjustment has been made to below a predetermined level.

5. The method according to any preceding claim, wherein the widefield ophthalmic imaging instrument (10) further comprises a polarised light source (14) arranged to generate linearly polarised light, the optical element (12) comprises a quarter-wave plate (100) arranged to generate non-linearly polarized light (L_{NL}) from the linearly polarised light (L_{LP}), and wherein an angle of rotation of the quarter-wave plate (100) about a propagation direction of the linearly polarised light (L_{LP}) is adjusted to set the respective state of polarization of the non-linearly polarized light (L_{I}) for acquiring each image of the plurality of images.

6. The method according to any preceding claim, wherein the determined adjustment, when made to the optical element (12), causes the ultra-widefield ophthalmic imaging instrument (10) to illuminate an imaging target being imaged by the ultra-widefield ophthalmic imaging instrument (10) with circularly polarised light.

7. The method of any preceding claim, wherein the non-linearly polarized light (L_{I}) has at least one wavelength between 400 nm and 570 nm.

8. The method according to any preceding claim, wherein the ultra-widefield ophthalmic imaging instrument (10) comprises at least one of an ultra-widefield fundus camera, an ultra-widefield scanning laser ophthalmoscope, or an ultra-widefield optical coherence tomography instrument.

9. A computer program (345) comprising instructions which, when executed by a processor (320) arranged to control a widefield ophthalmic imaging instrument (10), which is operable to image a region (22) of an ocular fundus (24) by illuminating the region (22) with non-linearly polarized light (L_{I}) whose state of polarisation is variable by adjustment of an optical element (12), cause the processor (320) to perform the method of any preceding claim.

10. A computer-readable storage medium (350) storing the computer program (345) according to Claim 9.

11. An ultra-widefield ophthalmic imaging instrument (10) operable to image a region (22) of an ocular fundus (24) by illuminating the region (22) with non-linearly polarized light (L_{I}) and detecting light (Lc) from the region (22), the ultra-widefield ophthalmic imaging instrument (10) comprising an optical element (12) which is adjustable to vary a state of polarization of the non-linearly polarized light (L_{I}), wherein the optical element (12) has been adjusted to be in a state of adjustment such that further adjustment of the optical element (12) to vary the state of polarization of the non-linearly polarized light (L_{I}) causes a degree of a variation in at least one of a local contrast or a local brightness across a representation of the region (22) in a subsequent image (30) acquired by the ultra-widefield ophthalmic imaging instrument (10) after the further adjustment to be at least as large as a degree of the variation in an initial image (40) acquired by the ultra-widefield ophthalmic imaging instrument (10) whilst the optical element (12) is in the state of adjustment.

12. The ultra-widefield ophthalmic imaging instrument (10) according to Claim 11, further comprising a polarised light source (14) arranged to generate linearly polarised light, wherein the optical element (12) comprises a quarter-wave plate (100) arranged to generate non-linearly polarized light (L_{NL}) from the linearly polarised light (L_{LP}), and wherein an angle of rotation of the quarter-wave plate (100) about a propagation direction of the linearly polarised light (L_{LP}) is adjustable to vary the state of polarization of the non-linearly polarized light (L_{I}).

13. The ultra-widefield ophthalmic imaging instrument (10) according to Claim 11 or Claim 12, wherein the state of adjustment of the optical element (12) is such that the region (22) is illuminated with circularly polarized light during imaging of the region (22) by the ultra-widefield ophthalmic imaging instrument (10).

14. The ultra-widefield ophthalmic imaging instrument (10) according to any of Claims 11 to 13, wherein the non-linearly polarized light (L_{I}) has at least one wavelength between 400 nm and 570 nm.

15. The ultra-widefield ophthalmic imaging instrument (10) according to any of Claims 11 to 14, comprising at least one of an ultra-widefield fundus camera, an ultra-widefield scanning laser ophthalmoscope, or an ultra-widefield optical coherence tomography instrument.
